(19) Europäisches Patentamt

European Patent Office

Office européen des brevets

(11) Veröffentlichungsnummer: **0 406 618 B1**

(12) **EUROPÄISCHE PATENTSCHRIFT**

(45) Veröffentlichungstag der Patentschrift: **01.06.94**

(21) Anmeldenummer: **90111621.0**

(22) Anmeldetag: **20.06.90**

(51) Int. Cl.5: **C08G 18/78**, C08G 18/80, C08G 18/76, C08G 18/30, C07C 275/62

(54) **Verfahren zur Herstellung von Harnstoff- und Biuretgruppen enthaltendem 1,5 Naphtylendiisocyanat.**

(30) Priorität: **04.07.89 DE 3921861**

(43) Veröffentlichungstag der Anmeldung:
**09.01.91 Patentblatt 91/02**

(45) Bekanntmachung des Hinweises auf die Patenterteilung:
**01.06.94 Patentblatt 94/22**

(84) Benannte Vertragsstaaten:
**AT BE CH DE ES FR GB IT LI NL SE**

(56) Entgegenhaltungen:
**EP-A- 0 325 115**
**FR-A- 2 272 113**
**US-A- 3 124 605**

**DATABASE WPIL Derwent Publications Ltd.,London, GB; DATABASE WPIL, accession no. 84-007510, week 8402; & JP- A - 58998327 (TAKEDA CHEMICAL IND. K.K.) 11.06.1983**

(73) Patentinhaber: **BAYER AG**

**D-51368 Leverkusen(DE)**

(72) Erfinder: **Grögler, Gerhard, Dr.**
**von-Diergardt-Strasse 48**
**D-5090 Leverkusen 1(DE)**
Erfinder: **Ruckes, Andreas, Dr.**
**Harderstrasse 13**
**D-5090 Leverkusen 3(DE)**
Erfinder: **Kopp, Richard, Dr.**
**Bilharzstrasse 15**
**D-5000 Köln 80(DE)**
Erfinder: **Hess, Heinrich, Dr.**
**Auf der Schildwache 13a**
**D-5000 Köln 80(DE)**
Erfinder: **Eiben, Robert, Dr.**
**Jakob-Böhme-Strasse 8**
**D-5000 Köln 80(DE)**
Erfinder: **Barnes, James Michael**
**Bechhausen 60**
**D-5632 Wermelskirchen 1(DE)**

**Beschreibung**

Die vorliegende Erfindung betrifft ein Verfahren zur Herstellung von Harnstoff- und Biuretgruppen aufweisendes 1.5-Naphthylendiisocyanat (NDI).

Die Herstellung von vernetzten Kunststoffen aus linearen, im wesentlichen Hydroxylgruppen aufweisenden Polyestern oder Polyethern mit einem Überschuß an organischen Diisocyanaten über die zur Reaktion mit den Hydroxylgruppen erforderlichen Menge ist seit langem bekannt. Bei dieser Reaktion tritt eine Verknüpfung der Polyester- oder Polyetherketten über Urethangruppen ein, und es entstehen lineare Gebilde, die an den Kettenenden freie Isocyanatgruppen enthalten. Das Molekulargewicht dieser sogenannten linearen isocyanatgruppenhaltigen Polyester oder Polyether ist um so größer, je kleiner der Überschuß an Diisocyanat über die zur Reaktion mit den Hydroxylgruppen erforderlichen Menge ist, und umgekehrt. Die so erhaltenen isocyanatgruppenhaltigen Polyester oder Polyether können im wesentlichen nach drei Verfahren in hochwertige, vernetzte Kunststoffe überführt werden.

Das erste Verfahren (deutsche Patentschrift 831 772) besteht in der Umsetzung der isocyanatgruppenhaltigen Polyester oder Polyether mit Glykolen. Bei dieser Reaktion tritt zunächst eine Verlängerung über Urethangruppen ein, wobei dann in zweiter Phase weitere Isocyanatgruppen mit den H-Atomen der Urethangruppen über Allophanatbindungen zur Vernetzung des Moleküls beitragen. Dieses Verfahren erlaubt eine Verarbeitung in flüssiger Phase und gestattet, nach dem Gießverfahren ohne Mitverwendung von Lösungsmitteln die verschiedenartigsten Formkörper herzustellen.

Das zweite Verfahren benutzt anstelle der Glykole Diamine, wobei eine Verlängerung des isocyanatgruppenhaltigen Polyesters oder Polyethers über zwei benachbarte Harnstoffgruppen eintritt, deren Wasserstoff-Atome mit noch vorhandenen Isocyanatgruppen unter Ausbildung von Biuretbindungen unter Vernetzung reagieren.

Das dritte Verfahren besteht in der Umsetzung der isocyanatgruppenhaltigen Polyester oder Polyether mit Wasser, wodurch eine weitere Verbindung von zwei Isocyanatgruppen durch Harnstoffverkettung erzielt wird. Auf diese Weise erhält man ein hochmolekulares Produkt. Auch hier reagieren die Wasserstoff-Atome der Harnstoffe mit überschüssigen Isocyanatgruppen, was die Bildung von Biureten als Querverbindungen bedingt. Da diese Biuretgruppierungen thermisch stabiler sind als die im ersten Verfahren genanntn Allophanatgruppen, zeigen die nach dem zweiten bzw. dritten Verfahren hergestellten Elastomere ein besseres mechanisches Eigenschaftsbild, was sich insbesondere im Hinblick auf Struktur, Elastizität, Druckverformungsrest und Abrieb bemerkbar macht. Das dritte Verfahren hat jedoch den Nachteil, daß bei der Reaktion der Isocyanatgruppen mit Wasser Kohlendioxid frei wird, so daß eine Verarbeitung des Materials in flüssiger Phase wegen der damit verbundenen Blasenbildung nicht möglich ist. Man ist daher gezwungen, einen technisch aufwendigen Arbeitsprozeß in Kauf zu nehmen, wobei man das schaumige Polyurethan-Material mittels hohem Druck unter Formgebung verpreßt.
(vgl. z.B. Kunststoff-Handbuch, Carl Hanser Verlag, 1966, Bd. VII, Seiten 270 - 271).

Die durch diese vielen Einzelschritte sehr aufwendige Fertigung erlaubt den Einsatz des Verfahrens nur für sehr anspruchsvolle Anwendungen. Ein großer Nachteil des Preßverfahrens ist außerdem, daß nur Plattenware oder geometrisch sehr einfach geformte Teile hergestellt werden können.

Andererseits weisen Harnstoffgruppen enthaltende Polyisocyanat-Polyadditionsprodukte, insbesondere Harnstoff aufweisende Polyurethane besondere hochwertige mechanische Eigenschaften auf, wobei die Verwendung von Wasser als KettenverläNgerer (anstelle von Diaminen) eine besonders einfach und preisgünstige Methode der Einführung von Harnstoffgruppen darstellt.

In der Patentliteratur wurden daher auch einige Verfahren beschrieben, bei denen für die Umsetzung von NCO-Gruppen enthaltenden Voraddukten (2-Stufenprozeß) oder von Reaktionsgemischen aus Polyisocyanaten mit hoch-und/oder niedermolekularen NCO-reaktiven Verbindungen (1-Stufenprozeß) Wasser als Kettenverlängerer verwendet wird. Die Verfahren sind z.B. in DE-OS 3 407 931, US-PS 4 416 844 sowie in DE-OS 3 725 198 dargelegt.

Das charakteristische Merkmal besteht bei diesem Verfahren darin, daß die Polyaddition in geschlossener Form stattfindet. Das bei der Reaktion des Polyisocyanats und Wasser sich bildende $CO_2$ bewirkt einen sehr hohen Druckaufbau und verbleibt daher zu einem gewissen Teil oder auch vollständig in gelöster Form im Polyadditionsprodukt. Nach Ablauf der für die Aushärtung nötigen Zeitspanne ist es möglich, die Formteile dem Werkzeug zu entnehmen, ohne daß es unter der Wirkung des gelösten $CO_2$'s zu einer Verformung kommt. Man erhält z. Teil blasenfreie massive Polyadditionsprodukte, die dann bei Raumtemperatur allmählich das gelöste $CO_2$ abgeben. Diese Verfahren sind jedoch technisch sehr aufwendig.

In der Patentschrift DE-OS 2 107 678 wurden die aufgeführten Nachteile obiger Verfahren umgangen, indem man die zur Biuret-Quervsrbindung benötigten Harnstoffgruppen bereits über das Polyisocyanat, insbesondere das 1,5-Diisocyanatnaphthalin, einführt. Dieses Verfahren ist dadurch gekennzeichnet, daß ein modifiziertes 1,5-Diisocyanat-naphthalin verwendet wird, welches 0,02 bis 0,5 Mol, vorzugsweise 0,1 bis 0,25 Mol Harnstoff- und Biuretgruppen pro Mol 1,5-Diisocyanat-naphthalin aufweist. Dieses modifizierte Isocyanat wird vorteilhaft durch Erwärmen von 1,5-Diisocyanatnaphthalin mit der entsprechenden Menge an tert. Alkoholen wie z.B. tert.-Butanol auf z.B. 130°C hergestellt.

Im Falle der Verwendung von tert.-Butylalkohol zur Herstellung des modifizierten Isocyanats wird das primär entstehende tert. Butylurethan des 1,5-Naphthylendiisocyanats thermisch gespalten, wobei Kohlendioxid und Isobuten entweicht. Bei Mitverwendung von Katalysatoren, wie z.B. Halogenwasseretoffsäuren oder Salzen aus stickstoffhaltigen Basen und anorganischen oder organischen Säuren, kann die Spalttemperatur wesentlich herabgesetzt werden, so daß man ein modifiziertes 1,5-Naphthylendiisocyanat definierterer Struktur erhält. Bei der entsprechenden Umsetzung dieses modifizierten 1,5-Naphthylendiisocyanats mit einem linearen Polyester oder Polyether entsteht dann zunächst das harnstoff- bzw. biuretgruppenhaltige NCO-Voraddukt, das anschließend mit (nieder- oder hochmolekularen) Diolen weiter verarbeitet werden kann. In dieser Phase können die Urethan- wie auch die bereits eingebauten Harnstoffgruppen mit noch überschüssigen Isocyanatgruppen unter Vernetzung des Moleküls weiterreagieren; wie die Vernetzung mittels Glykolen oder Diaminen verläuft auch diese Reaktion additiv. Das Verfahren stellt somit eine Kombination zwischen der Vernetzung mittels Wasser und Glykolen bzw. Polyolen dar, wobei die Nachteile der Wasservernetzung durch die im Polyisocyanat vorgebildeten Harnstoffgruppen ausgeschlossen sind. Die Vorteile des Verfahrens gegenüber dem bisher bekannten Verfahren bestehen darin, daß die Vernetzungsreaktion mit Diolen, insbesondere mit höhermolekularen Diolen (Molekulargewicht 500 - 6000) infolge der aktivierenden Wirkung der bereits im Polyisocyanat vorhandenen Harnstoff- bzw. Biuretgruppen rascher erfolgt, und daß daher in kurzer Zeit entformt werden kann. Die auf diese Weise hergestellten Kunststoffe sind kautschukelastisch und besitzen ein den wasservernetzten Polyurethanelastomeren (Verfahren 3) vergleichbares hochwertiges mechanisches Eigenschaftsbild.

Nachteilig bei diesem Verfahren ist jedoch, daß als "Wasser-Spender" tert. Alkohole (vorzugsweise tert. Butanol) verwendet werden. Die durch Reaktion mit dem Isocyanat zunächst sich bildenden tert. Alkylurethane sind insbesondere in Gegenwart von sauren Katalysatoren oberhalb gewisser Temperaturen unbeständig. Dabei entsteht ein Gasgemisch aus $CO_2$ und einem ungesättigten Kohlenwasserstoff. Diese Kohlenwassertoffe sind gasförmig, leicht flüssig und besitzen den Nachteil der leichten Entflammbarkeit. (Die einfache "Abfacklung" dieser Gase ist unter heutiger ökologischer Sicht nicht mehr möglich.) Sie müssen daher aus den Gasgemischen vom $CO_2$ getrennt bzw. isoliert werden. Dies ist jedoch mit einem hohen technischen Aufwand verbunden.

Gemäß JP-A 58098327 werden Isocyanat-Prepolymere, die Biuret-Strukturen aufweisen, dadurch hergestellt, daß dem Isocyanat vor der Umsetzung mit Wasser eine oberflächenaktive Substanz zugesetzt wird. In den Beispielen werden flüssige, aliphatische Isocyanate eingesetzt.

In der DE-OS 2 107 678 wird zwar darauf hingewiesen, daß neben Schwefelwasserstoff und Ameisensäure auch Wasser zur Modifizierung von 1,5-Naphthylendiisocyanat verwendet werden kann. Da weitere konkrete Angaben fehlen, wurden folgende Versuche durchgeführt.

Zunächst wurde die Umsetzung von $H_2O$ und 1,5-Naphthylendiisocyanat in Lösungsmittel (z.B. Ethylmethyl-keton, Dioxan, Chlorbenzol) vorgenommen, wobei die Reaktionstemperaturen von 100 auf 120 - 130°C erhöht wurden. In allen Fällen scheidet sich jedoch in kurzer Zeit ein Feststoff ab, der sich auch nach längerer Reaktionszeit nicht mehr auflöst. Es handelt sich hierbei um den aus 2 Mol 1,5-Naphthylendiisocyanat und 1 Mol Wasser gebildeter Naphthylendiisocyanat-harnstoff. Infolge seiner Schwerlöslichkeit reagiert diese Verbindung nur sehr träge mit OH-enthaltenden Komponenten ab. Nach dem Filtrieren und Einengen des Lösungsmittels erhält man unverändertes NDI zurück. Das so erhaltene NDI enthält keine Harnstoff- und Biuretgruppen.

In einem weiteren Versuch wurde daher ohne Lösungsmittel gearbeitet und die Reaktion von Wasser mit NDI (0,1 - 0,4 Mol Wasser pro 1 Mol NDI) oberhalb der Schmelztemperatur von NDI (130 - 140°C) durchgeführt. Hierbei wurde beobachtet, daß ein beträchtlicher Anteil der Wassermenge im Kühlsystem oder an kälteren Glaswandungen des Reaktionsgefäßes kondensiert. Dieser Anteil entzieht sich somit der Reaktion mit NDI. Eine genaue Dosierung der Wassermenge ist daher nicht möglich. Eine weitere Erschwerung der Reaktionsführung liegt darin, daß sich eine infolge der starken Sublimationsneigung von NDI zunehmende Ablagerung von NDI an den kälteren Glaswandungen bemerkbar macht. Es bilden sich alsbald dicke NDI-Krusten und $NDI/H_2O$-Folgeprodukte wie z.B. NDI-Polyharnstoffe. Auch dieses Verfahren ist zur Modifizierung von NDI nicht geeignet.

Der vorliegenden Erfindung lag daher die Aufgabe zugrunde, eine technisch einfache Herstellungsmethode für Harnstoff- und Biuretgruppen aufweisendes 1,5-Naphthylendiisocyanat (NDI) zu erarbeiten.

Es wurde nun überraschend gefunden, daß die Modifizierung von NDI dann ohne technischen Aufwand abläuft, wenn die zur Reaktion erforderliche Wassermenge in Gegenwart von wenig org. Lösungsmittel vorliegt. Das Lösungsmittel soll dabei einen Siedepunktsbereich von 80 bis 140°C aufweisen und mit Wasser mischbar sein oder zumindest im Siedepunktsbereich ein azeotropes Gemisch mit Wasser ergeben.

Gegenstand der Erfindung ist somit ein Verfahren zur Herstellung von Harnstoff-und Biuretgruppen aufweisendes NDI, dadurch gekennzeichnet, daß in geschmolzener Form vorliegendes NDI mit 0,02 bis 0,5, vorzugsweise 0,1 bis 0,25 Mol Wasser (pro Mol NDI) im Gemisch mit der 1 bis 10-fachen, vorzugsweise 2- bis 5-fachen Menge eines mit Wasser mischbaren oder zumindest mit Wasser im Siedebereich ein azeotropes Gasgemisch bildenden organ. Lösungsmittels umgesetzt wird. Die Umsetzungstemperatur beträgt vorzugsweise 130 bis 160° C besonders bevorzugt 130° bis 140° C. Eine Ausführungsform des Verfahrens ist dadurch gekennzeichnet, daß ein Lösungsmittel verwendet wird, dessen Siedepunkt im Bereich von 80 bis 140°C, vorzugsweise 100 bis 120°C liegt.

Das nach dem erfindungsgemäßen Verfahren hergestellte Harnstoff- und Biuretgruppen aufweisende NDI kann vorteilhaft zur Fertigung von Polyurethan- oder Polyurethanharnstoff-Elastomeren eingesetzt werden.

Dabei wird Harnstoff- und Biuretgruppen aufweisendes NDI umgesetzt mit:

A) Verbindungen mit mindestens zwei gegenüber Isocyanatgruppen reaktionsfähigen Gruppen des Molgewichtes 500 bis 10 000,

und gegebenenfalls

B) organischen Verbindungen mit gegenüber Isocyanatgruppen reaktionsfähigen Wasserstoffatomen des Molekularbereichs von 60 bis 499,

C) gegebenenfalls unter Mitverwendung von den aus der Polyurethanchemie an sich bekannten Hilfs- und Zusatzstoffen,

nach dem Einstufen- oder Prepolymerverfahren unter Einhaltung einer Isocyanatkennzahl von 70 bis 130.

Unter Einstufenverfahren ist eine Arbeitsweise zu verstehen, gemäß welcher die Komponenten A) und gegebenenfalls B) und C) zu einer "Reaktionskomponente" vereinigt werden, die dann einstufig mit dem Harnstoff- und Biuretgruppen enthaltenden NDI in geschmolzener Form zur Reaktion gebracht wird.

Unter Prepolymerverfahren ist eine Arbeitsweise zu verstehen, gemäß welcher die Polyisocyanatkomponente (modifiziertes NDI) mit mindestens einem Teil der Komponente A) und gegebenenfalls mit einem Teil der Komponente B) und C) zu einem Isocyanatgruppen aufweisenden Prepolymer umgesetzt wird. Dieses wird anschließend mit einem Gemisch aus den restlichen Mengen der Komponente A) und gegebenenfalls B) und C) zur Reaktion gebracht.

Eine bevorzugte Anwendung des Harnstoff- und Biuretgruppen enthaltenden NDI für die Herstellung von Polyurethan- oder Polyurethanharnstoff-Formteilen besteht darin, daß das erfindungsgemäß modifizierte NDI vor der Verwendung mittels geeigneter Mühlen fein gemahlen (Teilchengröße 1 - 50 $\mu$.m, vorzugsweise 3 - 20 $\mu$.m) und da so erhaltene Isocyanat-Pulver als Feststoff einsetzt.

In der Praxis wird so verfahren, daß das Feststoffisocyanat mittels eines geeigneten Rührwerks in der entsprechenden flüssigen Komponente (vorzugsweise A) fein verteilt wird. Die so erhaltene feinteilige Suspension ist infolge der Schwerlöslichkeit des Isocyanats bei Temperaturen von 10 bis 50°C längere Zeit lagerstabil. Die Verfestigung (Polyaddition) erfolgt dann bei erhöhter Temperatur, wobei sich eine Verfestigungstemperatur von 100 bis 150°C als sehr günstig erweist.

Besonders bevorzugt zur Anwendung Kommt ein Verfahren, bei dem die Suspension des Feststoffdiisocyanats in der flüssigen Reaktivkomponente mit geringen Mengen eines aliphatischen Diamins vermischt wird. Hierbei bildet sich auf der Oberfläche der Isocyanatteilchen eine dünne Polyharnstoffhülle, die als Diffusionssperrschicht wirkt. Man erreicht bei diesem Verfahren, daß solche "desaktivierten oder retardierten" Suspensionen bei Raumtemperatur und selbst bei erhöhten Temperaturen bis 80 - 100°C lagerstabil bleiben. Man erhält Einkomponentensysteme. Die Verfestigung erfolgt dann zu einem beliebigen Zeitpunkt durch Hitzestoß, wobei das Feststoffdiisocyanat zum Teil aufschmilzt und in gelöster Form mit der NCO-aktiven Komponente reagiert. Die Verfestigungstemperatur liegt in einem Bereich von 120 bis 150°C.

Dieses Verfahren ist in der US-Patentschrift US 4 483 974 bzw. EP 103 323 beschrieben.

Die erfindungsgemäße Herstellung von Harnstoff- und Biuretgruppen aufweisendem NDI gelingt ohne technischen Aufwand. Sie wird wie folgt durchgeführt:

Zu 1,0 Mol NDI-Schmelze wird bei einer Temperatur von 130 - 140°C unter Rühren ein Gemisch aus Wasser und organischem Lösemittel zugetropft. Der Wasseranteil beträgt 0,05 bis 0,5 Mol, vorzugsweise 0,1 bis 0,25 Mol/Mol NDI. Ein wesentliches Merkmal der Erfindung besteht darin, daß Wasser im Gemisch mit organischen Lösungsmitteln verwendet wird. Diese Lösemittelanteile sind im Vergleich zu dem Gesamtreaktionsansatz gering. Wird dagegen in hoher Lösungsmittelverdünnung gearbeitet, so treten die bereits erwähnten Nachteile der vorzeitigen Abscheidung von unlöslichen oligomeren NDI-Harnstoffdiisocyanaten auf. Darüber hinaus muß nach Beendigung der Reaktion das gesamte Lösungsmittel destillativ entfernt

4

werden, was einen zusätzlichen technischen Aufwand bedeutet.

Im Sinne der Erfindung wird daher nur die 1- bis 10-fache vorzugsweise 1- bis 5-fache Menge an Lösungsmittel, berechnet an einem Gewichtsteil Wasser, verwendet.

Hierbei werden Lösungsmittel eingesetzt, die in einem Siedepunktbereich von 80 bis 140°C; vorzugsweise 80 bis 120°C, liegen und gegenüber Wasser ein gewisses Lösungsvermögen aufweisen. Beispielhaft sind zu nennen: Dioxan, Acetonitril, Diethylcarbonat, Ethylmethylketon, Methylisobutylketon, Ethylenglykoldimethylether. Dieses Verfahren ist bevorzugt.

Es können aber auch Lösemittel Verwendung finden, die mit Wasser nur wenig mischbar sind. Sie sollen aber im Siedepunktbereich mit Wasser ein azeotropes Wasser/Lösungsmittel-Gemisch ergeben. Beispielhaft seien genannt: Benzol Toluol, Xylol, Chlorbenzol. Dieses Verfahren ist weniger bevorzugt.

Während der Zugabe des Wasser/Lösungsmittelgemisches zu der NDI-Schmelze bei 130 - 140°C erfolgt kontinuierlich Kohlendioxid-Abgabe, wodurch eine sichere Kontrolle des Reaktionsansatzes gewährleistet wird. Nach Beendigung der $CO_2$-Entwicklung wird unter Anlegen von Vakuum das im Reaktionsgemisch noch gelöste Kohlendioxid und das Lösungsmittel entfernt. Man läßt das noch flüssige Reaktionsprodukt erkalten und kann dieses so modifizierte NDI gleich oder nach eventueller Mahlung für die Herstellung von Polyurethanen verwenden.

Das erfindungsgemäß hergestellte modifizierte NDI wird nunmehr mit einer Verbindung mit mindestens zwei gegenüber Isocyanatgruppen reaktionsfähigen Gruppen umgesetzt (Komponente A). Es handelt sich hierbei um eine Verbindung, die vorzugsweise Hydroxyl- und/oder Aminogruppen als Endgruppen aufweist und ein Molekulargewicht von 500 bis 10 000, vorzugsweise 1000 bis 3000 besitzt.

Als Dihydroxylverbindungen kommen dabei insbesondere die an sich bekannten Polyester und Polyether infrage. Als Polyether seien solche genannt, die durch Umsetzung von Alkylenoxiden wie Ethylenoxid, Propylenoxid, Epichlorhydrin oder Tetrahydrofuran mit sich selbst oder mit Startverbindungen erhalten werden. Als Startverbindungen seien beispielsweise Wasser, Polyole oder Polyamine genannt. Als Polyole kommen z.B. Ethylenglykol, Propylenglykol, Butandiol infrage, während als Polyamine Ethylendiamin, Hexamethylendiamin beispielsweise genannt sein sollen. Polyether wie sie erfindungsgemäß verwendet werden können, finden sich z.B. in der britischen Patentschrift 769 091 und in der deutschen Patentschrift 974 371 sowie in der US-Patentschrift 2 948 691 und in der US-Patentschrift 2 929 800.

Als höhermolekulare Polyaminoverbindungen mit aliphatischen Aminogruppen werden z.B. solche eingesetzt, wie sie durch reduktive Aminierung von Polyoxyalkylenglykolen mit Ammoniak nach BE-PS 634 741 und US-PS 3 654 370 erhalten werden. Weitere höhermolekulare Polyoxyalkylen-Polyamine können nach Methoden, wie sie in der Firmenschrift "Jeffamine, Polyoxypropylene Amines" von Texaco Chemical Co. 1978, aufgezählt werden, hergestellt werden, beispielsweise durch Hydrierung von cyanethylierten Polyoxypropylenglykolen (DE-OS 1 193 671), durch Aminierung von Polypropylenglykolsulfonsäureestern (US-PS 3 236 895), durch Behandlung eines Polyoxyalkylenglykols mit Epichlorhydrin und einem primären Amin (FR-PS 1 466 708) oder durch Umsetzung von NCO-Prepolymeren mit Hydroxylgruppen aufweisenden Enaminen, Aldiminen oder Ketiminen und anschließende Hydrolyse gemäß DE-OS 2 546 536. Geeignete höhermolekulare aliphatische Di- und Polyamine sind auch die nach DE-OS 2 948 419, DE-OS 3 039 600, DE-OS 3 112 118, der EP-A 61 627, EP-A 71 132 und EP-A 71 139 durch alkalische Hydrolyse von NCO-Prepolymeren mit Basen über die Carbamatstufe zugänglichen Polyamine.

Die linearen Polyester, die vorzugsweise als Ausgangsmaterial für die vorliegende Erfindung angewandt werden, werden am zweckmäßigsten aus im wesentlichen gesättigten aliphatischen Produkten hergestellt. Als Säuren seien hier erwähnt: Malonsäure, Bernsteinsäure, Adipinsäure, Methyladipinsäure, Maleinsäure, Kohlensäure, Dihydromuconsäure, Thiopropionsäure, Diethyletherdicarbonsäure, Sebacinsäure, Korksäure und höhere Dicarbonsäuren. Als Glykole seien angeführt:
Ethylenglykol, 1,2-Propylenglykol, 1,3-Propylenglykol, 1,3-Butylenglykol, 1,4-Butylenglykol, 1,6-Hexandiol, Methylhexandiol-1,6, 1,4-Dimethyl-1,3-propylenglykol. Oxycarbonsäuren können ebenfalls bei der Herstellung von Polyestern benutzt werden, vorausgesetzt jedoch, daß die Tendenz zur Polykondensation stärker ist als die zur Ringbildung, und daß genügende Mengen Glykol vorhanden sind, um die Bildung von Hydroxylendgruppen zu gewährleisten. Außerdem können Mischungen verschiedener Säuren und Glykole benutzt werden, wodurch man gemischte Polyester erhält. Anstelle der Säuren und Glykole kann man anders bifunktionelle Reaktionspartner in kleineren Mengen ebenfalls benutzen. Als Beispiel seien Verbindungen erwähnt, die aromatische Kerne enthalten, wie Phthalsäure oder Terephthalsäure, aromatische oder aliphatische Diamine, wie z.B. Phenylendiamine, Naphthylendiamine, Piperazin, Ethylendiamin, sowie auch Aminoalkohole, wie z.B. Aminopropanol oder Oxethylanilin. Die Polykondensation der Reaktionskomponenten findet durch Erhitzen bei 100 bis 250°C statt. Die OH-Zahl der Polyester soll möglichst zwischen 20 und 100 liegen, am besten zwischen 40 und 60, das Molekulargewicht beträgt vorteilhat dann 500 - 6000. Bevor die Reaktion mit dem Harnstoff- bzw. Biuretgruppen enthaltenden Isocyanat stattfindet, sollten die Polyester zweckmäßig von unter Umständen anhaftender Feuchtigkeit befreit werden. Dies geschieht z.B. durch Erhitzen auf 100 - 150°C im Vakuum oder mittels Durchleiten inerter Gase bei der gleichen Temperatur. Unter den Säuren bevorzugt man Adipinsäure und unter den Glykolen Ethylenglykol und 1,2-Propylenglykol als Polyesterkomponenten.

Es kann oft erfindungsgemäß bevorzugt sein, Polycaprolactone als Ausgangsmaterial einzusetzen, z.B. solche wie sie in den US-Patentschriften 3 169 945, 2 914 556, 2 890 208, 2 878 236 oder in der britischen Patentschrift 859 645 beschrieben werden.

Eine bevorzugte Anwendung beinhaltet die Herstellung von Gießelastomeren, in denen als NCO-reaktive Komponenten Polyesterpolyole bzw. mit aromatischen Amingruppen terminierte Polyester eingesetzt werden. Die Polyesterpolyamine werden vorzugsweise nach der E-AS 0 219 035 durch Hydrolyse von endständigen Isocyanatgruppen aufweisenden Verbindungen erhalten. Bei diesem Verfahren werden insbesondere zwei oder drei Hydroxylgruppen aufweisende Polyester zu NCO-Prepolymeren umgesetzt und in einem zweiten Schritt die Isocyanatgruppen durch Hydrolyse in eine Aminogruppe überführt. Man erhält Elastomere mit hervorragendem Wärmestand und ausgezeichneten mechanischen Eigenschaften.

Selbstverständlich können Mischungen der obengenannten Verbindungen mit mindestens zwei gegenüber Isocyanaten reaktionsfähigen Wasserstoffatomen, z.B. Mischungen von Polyethern und Polyestern, eingesetzt werden.

Als Polyole kommen gegebenenfalls auch niedermolekulare Kettenverlängerer oder Vernetzer in Frage (Komponente B). Bei diesen Kettenverlängerern oder Vernetzern handelt es sich insbesondere um mindestens zwei funktionelle Verbindungen, welche an aliphatische und/oder cycloaliphatische Gruppen gebundene Hydroxylgruppen und Molgewichte zwischen 60 und 499 aufweisen. Bevorzugt sind dabei niedermolekulare Diole mit an aliphatische oder cycloaliphatische Gruppen gebundene Hydroxylgruppen.

Als Beispiele für derartige Verbindungen seien genannt: Ethylenglykol, Diethylenglykol, Triethylenglykol, Butandiol-1,4, Butandiol-2,3, Pentandiol-1,5, Hexandiol-1,6, Neopentylglykol, 1,4-Bis-hydroxyethyl-cyclohexan, 1,4-Dihydroxycyclohexan, Terephthalsäure-bis($\beta$-hydroxyethyl)ester, 1,4,3,6-Dianhydrohexite, 1,4-Monoanhydrotetrite, Propylenglykol, Tetrapropylenglykol, Bis-2-hydroxyethyl-hydrochinon, Bis-(2-hydroxyethyl)-resorcin. Als mehrwertige Verbindungen seien genannt: Trimethylolpropan, Trimethylolethan, Hexantriol-1,2,6.

Es sind auch Diole mit zusätzlichen Gruppen einsetzbar, z.B. Adipinsäure-bis-(2-hydroxyethyl)-ester, Trephthalsäure-bis-(2-hydroxyethyl)-ester, Diol-urethane, Diolharnstoffe oder Polyole, welche Sulfonat- und/oder Phosphonatgruppen enthalten, z.B. 1,6-Hexamethylen-bis-(2-hydroxyethylenurethan), 4,4'-Diphenylmethan-bis-(2-hydroxyethylharnstoff) oder das Addukt von Na-Bisulfit an Butandiol-1,4, bzw. dessen Alkoxylierungsprodukte. Weitere niedermolekulare Verbindungen werden ausführlich in der DE-A 2 854 384 beschrieben.

Als Kettenverlängerer können niedermolekulare aromatische Diamine insbesondere dann eingesetzt werden, wenn das erfindungsgemäße Harnstoff- und Biuretgruppen aufweisende NDI als fein verteiltes Feststoffdiisocyanat vorliegt und die NDI-Teilchenoberfläche gemäß EP 103 323 mittels einer Diffusionssperrschicht gegenüber dem Angriff NCO-reaktiver Gruppen "retardiert" ist.

Unter aromatischen Polyaminen sollen auch solche Amine verstanden werden, welche die Aminogruppe an heterocyclische Reste mit aromatischem Charakter gebunden enthalten. Als aromatische Polyamine sind z.B. geeignet: p-Phenylendiamin, 2,4-/2,6-Toluylendiamine, Diphenylmethan-4,4'- und/oder - 2,4'- und/oder -2,2'-diamine, 3,3'-Dichlor-4,4'-diaminodiphenylmethan, 3-($C_1$-$C_8$)-Alkyl-4,4'-diaminodiphenylmethane, die 3,3'-Di-($C_1$-$C_4$)-4,4Ä-diaminodiphenylmethyl sowie die 3,3', 5,5'-Tetra-($C_1$-$C_4$)-alkyl-4,4'-diphenylmethane, die 4,4'-Diaminodiphenyl-sulfide, -sulfoxide oder -sulfone, Ethergruppen aufweisende Diamine gemäß DE-A 1 770 525 und 1 809 172 (US-PS 3 654 364 und 3 736 295), gegebenenfalls in 5-Stellung substituierte 2-Halogen-1,3-phenylendiamine (DE-AS 2 001 772, 2 025 896 und 2 065 869), Bis-anthranilsäureester (DE-A 2 040 644 und 2 160 590), 2,4-Diaminobenzoesäureester nach DE-A 2 025 900, sowie durch eine oder zwei ($C_1$-$C_4$-Alkylgruppen substituierte Toluylendiamine. Besonders bevorzugt sind 3,5-Diethyl-2,4- und/oder -2,6-diaminotoluol (besonders ihre technischen (80/20)- oder (65/35)-Isomerengemische), unsymmetrisch tetraalkylsubstituierte Diaminodiphenylmethane, z.B. 3,5-Diethyl-3'-5'-diisopropyl-4,4'-diaminodiphenylmethan und ihre Isomerengemische entsprechend DE-A 2 902 090, 4,4'-Diaminobenzanilid sowie 3,5-Diaminobenzoesäure-($C_1$-$C_4$)-alkylester, 4,4'- und/oder 2,4'-Diamino-diphenylmethan, sowie Napthylen-1,5-diamin.

Niedermolekulare aromatische Diamine als Kettenverlängerer sind jedoch weniger bevorzugt.

Die Umsetzungsgeschwindigkeit der Komponenten kann durch Zugabe von organischen Basen wie tert. Aminen oder durch Metallsalze organischer Säuren beschleunigt werden. Von ersterer Gruppe seien z.B. genannt: Hexahydrodimethylanilin, Methylpiperazin, Dimethylpiperazin, Tribenzylamin, Tetramethyldiaminodipropylether.

Ähnliche Wirkungen werden erzielt, wenn man Polyester anwendet, die basischen Reaktionsgruppen in chemischer Bindung enthalten. Von den Metallsalzen organischer Säuren seien z.B. genannt: Zinn(II)-ethylhexoat oder Dibutyl-zinn-dilaurat.

Weichmacher, Farb- und Füllstoffe können ohne Schwierigkeiten während jeder Stufe der Vorbereitung zugegeben werden. Als Weichmacher eignen sich z.B. Phthalsäureester und organische Sulfonamide. Besonders günstig sind Schwefel enthaltende Weichmacher, wie z.B Methylen-bis-thioglykolsäurebutylester. Wie bei Naturkautschuk bewirkt ein Teil der Füllstoffe eine Verbesserung der mechanischen Eigenschaften der neuen kautschuk-elastischen Produkte. Dies trifft z.B. auf Tiandioxid, Siliciumdioxid, Bentonit, Calciumsilikat und Ruß zu. Die Glasfasern werden z.B. entweder in den Polyestern oder den Polyethern oder in das Harnstoff- bzw. Biuretgruppen enthaltenden NCO-Voraddukt eingearbeitet. Das hochwertige mechanische Eigenschaftsbild und die hervorragende Beständigkeit gegenüber Einflüssen von organischen Lösungsmitteln und Ölen eröffnet den beschriebenen kautschuk-elastischen Kunststoffen ein breites Einsatzgebiet. Als Beispiele seien genannt: Walzenbeläge, elastische Bauteile für Maschinen, Dichtungen, Puffer, Faltenbälge, Beläge für Kugelmühlen, Schuhe oder Teile von Schuhen, Bälle, Zylinder.

Nähere Einzelheiten der Erfindung werden in den nachfolgenden Beispielen beschrieben.

Beispiele

1. Vergleichsbeispiel (nicht erfindungsgemäß)

Eine Mischung von 210 g (1,0 Mol) 1,5-Naphthylendiisocyanat ( NDI) und 210 g Methyl-ethyl-keton wird unter Rühren auf 90 bis 100° erwärmt. Man erhält eine klare homogene Lösung von NDI in dem genannten Lösungsmittel. Nun werden der Lösung 3,6 g (0,2 Mol) Wasser tropfenweise zugesetzt. Bereits nach kurzer Zeit beginnt eine kristalline Abscheidung, die sich nach Isolierung durch Filtration als NDI-Harnstoffdiisocyanat (NCO = 20,7% berechnet = 21.3%) erweist. Diese Substanz ist in allen organischen Lösungsmitteln äußerst schwer löslich. Auch in obigem Ansatz erfolgt auch nach längerer Reaktionszeit bei 90 bis 100° keine Lösung. Man erhält eine Supension von NDI-Harnstoffdiisocyanat in der NDI/Methyl-ethyl-keton-Lösung.Die weitere Aufarbeitung ist aufwendig, da unter ständiger Temperaturerhöhung das entsprechende Lösungsmittel abdestilliert werden muß. Schließlich erhält man eine Suspension von NDI-Harnstoffdiisocyanat in der lösungsmittelfreien NDI-Schmelze als inhomogenes Endprodukt.

2. Vergleichsbeispiel (nicht erfindungsgemäß)

210 g (1,0 Mol) NDI (Fp: 127°C) werden auf eine Temperatur von 130 - 140° erwärmt. Nach einiger Zeit (ca. 1 Stunde) kann zu dem nun aufgeschmolzenen Polyisocyanat 3,6 g (0,2 Mol) Wasser tropfenweise zugesetzt werden.

Hierbei verdampft jedoch ein gewisser Wasseranteil und kondensiert an den kälteren Stellen des Reaktionsgefäßes. Auch noch nicht umgesetztes $H_2O$ tritt gasförmig heraus und kondensiert. (zum Teil auf dem Rückflußkühler, zum Teil auf der Glaswand). Mit zunehmender Reaktionsdauer sublimiert aber auch NDI in starken Maße. Auch hier erfolgt allmählich Abscheidung eines NDI-Sublimates in Form eines verfilzten Überzuges auf der "kalten" Glaswand. Es kommt dort zu einer Umsetzung mit dem kondensierten Wasser, wobei sich völlig unlöslicher NDI-Polyharnstoff bildet. Dieser löst sich allmählich von der Glaswand und fällt in das Reaktionsgefäß.

Dieses Verfahren ist daher nicht brauchbar. Es verläuft unkontrollierbar und eine genaue Wasserdosierung ist nicht möglich.

Nach Beendigung der Kohlendioxid-Entwicklung erhält man ein Endprodukt, das noch in mehr oder weniger großen Anteilen NDI-Polyharnstoff als völlig unlöslichen Bestandteil enthält.

Beispiel 3

Herstellung des erfindungsgemäßen Harnstoff- und Biuretgruppen enthaltenden 1.5-Naphthylendiisocyantes

2100 g (10,0 Mol) NDI werden in einem Dreihalskolben, versehen mit Rührer, Thermometer und Rückflußkühler, auf 130 bis 140° erhitzt. Nach Aufschmelzen des Isocyanates wird ein Gemisch von 27 g (1,5 Mol) Wasser und 81 g Dioxan (Verhältnis Wasser/Dioxan = 1:3) innerhalb von 1 bis 2 Stunden zugetropft. Es erfolgt keine sichtbare Abscheidung von Wasser an den kälteren Glaswänden des Reaktionsgefäßes. Auch kann durch diese Maßnahme eine Sublimation von NDI unterbunden werden, da das heiße Lösungsmittel Dioxan auch in geringen Mengen eine Abscheidung von NDI an den Glaswänden verhindert.

Die Reaktion von Wasser mit NDI kann durch Kontrolle der $CO_2$-Gasentwicklung verfolgt werden. Nach ca. 4 Stunden werden bei 130 bis 140°C ca. 34,5l $CO_2$ entwickelt. Danach wird bei gleichbleibender Temperatur mittels $H_2O$-Strahlvakuum das noch gelöste $CO_2$-Gas und das Lösungsmittel Dioxan entfernt und der noch flüssige Kolbeninhalt auf eine kalte Unterlage gegossen. Nach Erkalten der Schmelze kann das modifizierte NDI leicht zerkleinert werden. Der NCO-Gehalt beträgt 31.8%.

Bei diesem Verfahren treten die im Vergleichsbeispiel 2 erwähnten Nachteile nicht auf.

In nachstehender Tabelle werden einige Harnstoff- bzw. Biuretgruppen enthaltende Isocyanate angegeben, die in oben beschriebener Weise hergestellt wurden.

| Produkt | Mol 1.5-Naphthylendiisocyanat | Mol/g $H_2O$ | Dioxan (g) | % NCO |
|---------|-------------------------------|--------------|------------|-------|
| A | 1,0 | 0,1/1,8 g | 5,4 | 34,6 |
| B | 1,0 | 0,15/2,7 | 8,1 | 31,8 |
| C | 1,0 | 0,2/3,6 | 18,0 | 29,4 |
| D | 1,0 | 0,3/5,4 | 27,0 | 26,5 |
| E | 1,0 | 0,4/7,2 | 27,0 | 24,5 |

Beispiel 4

200 g eines Ethylenglykol-Adipinsäurepolyesters mit der OH-Zahl 56 (Molgewicht: 2000) werden bei 130° und 15 mm Druck entwässert. Anschließend trägt man unter Rühren 57,5 g des modifizierten Isocyanates C (gemäß Beispiel 3, NCO = 29,4%) ein. Man beobachtet zunächst ein Abfallen der Reaktionstemperatur auf ca. 120°C. Nach 10 - 15 Minuten ist die Temperatur wieder auf ca. 128°C gestiegen, wonach man 165 g des oben genannten Polyesters in geschmolzener Form einrührt und den Reaktionsansatz in auf 110°C erwärmte Formen gießt. Bei einer Ausheiztemperatur von 120°C kann der Probekörper nach ca. 1 Stunde entformt werden. Das PUR-Elastomer wird noch 5-8 Stunden bei 110 - 120°C nachgetempert. Nach einwöchiger Lagerung bei Raumtemperatur wurden folgende mechanische Werte gemessen.

| Shore Härte A | | Shore A | 70 |
|---------------|---|---------|-----|
| Belastung | bei 100% bei 300% | MPa MPa | 2,5 6,5 |
| Zugfestigkeit | | MPa | 42,3 |
| Bruchdehnung | | % | 57,0 |
| Weiterreißfestigkeit | | KN/m | 52,6 |
| Stoßelastizität | | % | 42 |

Beispiel 5

200 g eines gemäß Beispiel 4 entwässerten Ethylenglykol-Adipinsäurepolyesters mit der OH-Zahl 56 werden bei einer Temperatur von 60 bis 80°C mit 27,7 des modifizierten Isocyanates B (gemäß Beispiel 3, NCO = 31,8%) homogen vermischt. Das modifizierte NDI wurde vorher mit einer geeigneten Mahlvorrichtung (z.B. Kugel-Fliehkraftmühle) auf eine Teilchengröße von 20 bis 50 μm gebracht. Man erhält eine Suspension des Isocyanates in dem Polyester. Die Lagerstabilität dieser Suspension beträgt bei dieser Temperatur mehrere Stunden, so daß keinerlei Schwierigkeiten bei der weiteren Verarbeitung auftreten. Nach kurzem Entgasen wird der Ansatz in eine auf 110°C erwärmte Form gegossen und bei 130 bis 150°C ausgeheizt. Nach ca. 1 Stunde kann entformt und der Probekörper noch 3 bis 4 Stunden bei 120°C nachgetempert werden.

Nach diesem Verfahren wurden folgende höhermolekulare HO-Verbindungen verarbeitet. Die Molekulargewichte liegen bei 2000 (OH-Zahl: 56).

a. Polyester aus Adipinsäure und Ethylenglykol (gemäß Beispiel 4 und 5).

b. Polyester aus Adipinsäure und einem Gemisch aus Ethylenglykol und Butandiol-(1,4) (Mol-Verhältnis: 1:1).

c. Polyester aus Adipinsäure und einem Gemisch aus Ethylenglykol, Butandiol-(1,4) und Hexandiol-(1,6) (Mol-Verhältnis: 1:1:1).

d. Polyester aus Adipinsäure und Hexandiol-(1,6).

e. Polyester aus Kohlensäure und Hexandiol-(1,6). (Hergestellt durch Reaktion von Hexandiol-(1,6) mit Diphenylcarbonat.)

f. Polytetrahydrofuran.

Die in folgender Tabelle zusammengefaßten mechanischen Werte sind nach ca. einwöchiger Lagerung der Elastomeren gemessen.

| | | a | b | c | d | e | f |
|---|---|---|---|---|---|---|---|
| Shore Härte A | | 70 | 75 | 78 | 72 | 73 | 75 |
| Belastung | bei 100% (MPa) | 3,0 | 3,5 | 3,5 | 2,8 | 4,0 | 3,9 |
| | bei 300% (MPa) | 6,5 | 6,4 | 6,4 | 6,1 | 8,6 | 8,4 |
| Zugfestigkeit (MPa) | | 45,5 | 27,5 | 28,7 | 23,9 | 30,2 | 37,5 |
| Bruchdehnung (%) | | 680 | 570 | 500 | 565 | 580 | 380 |
| Weiterreißfestigkeit KN/m | | 75,6 | 35,8 | 32,7 | 35,7 | 39,5 | 27,5 |
| Stoßelastizität (%) | | 45 | 47 | 40 | 48 | 50 | 61 |

Beispiel 6

200 g eines Polyesters aus Adipinsäure und Ethylenglykol (gemäß Beispiel 4) werden bei 60 bis 80°C mit den angegebenen modifizierten NDI-Typen B, C,D und E (gemäß Beispiel 3) homogen vermischt. Das NCO/OH-Verhältnis beträgt hierbei 1,05.Auch hier wird ein feingemahlenes NDI-Produkt verwendet. (Teilchengröße: 20-50 µm). Vor der Zugabe des Pulvers werden jedoch 0,7 g des aliphatischen Diamins Isophorondiamin (5-Amino-3-(aminomethyl)-1,1,3-trimethyl-cyclohexan) der Polyesterschmelze zugesetzt. Durch diese Maßnahme wird erreicht, daß das zugefügte Polyisocyanat-Pulver nach der Reaktion mit dem aliphatischen Diamin JPDA in desaktiviertem (retardiertem) Zustand vorliegt.

(gemäß EP 103 323, Diffusions-Sperrschicht))
Die erhaltenen Polyester/Polyisocyanat Suspensionen sind nun bei Raumtemperatur oder gering erhöhter Temperatur lagerstabil. Die weitere Verarbeitung kann dann nach beliebigem Zeitpunkt durch Hitzestoß (130-150°C) erfolgen. Nach bereits beschriebener Weise erhält man Elastomere, die nach einwöchiger Lagerung bei Raumtemperatur folgendes mechanisches Wetterniveau zeigen.

| | B 27,7 g/ 200 g PE | C 30,0 g/ 200 g PE | D 33,3 g/ 200 g PE | E 36,6 g/ 200 g PE |
|---|---|---|---|---|
| Shore-Härte A | 73 | 74 | 77 | 79 |
| Belastung bei 100% MPa | 2,8 | 2,9 | 3,6 | 3,8 |
| Zugfestigkeit MPa | 39,6 | 38,7 | 37,8 | 25,8 |
| Bruchdehnung % | 650 | 650 | 650 | 720 |
| Weiterreißfestigkeit KN/m | 65,6 | 60,3 | 58,5 | 59,0 |
| Stoßelastizität % | 46 | 45 | 45 | 40 |

Beispiel 7

Zu 200 g eines mit aromatischen $NH_2$-Gruppen terminierten Polyethers (NH-Zahl 47), der durch alkalische Hydrolyse eines NCO-Prepolymeren aus einem Polypropylglykolether (OH-Zahl: 56, Molgewicht = 2000) und 2,4-Diisocyanatotoluol erhalten wurde, werden bei 60-80°C 1,5 g Isophorondiamin (IPDA) zugesetzt. Danach werden 26,7 g des Produkts B (vgl. Beispiel 3) in gemahlener Form eingerührt. Die Verarbeitung der entstandenen Suspension ist problemlos, da infolge der gebildeten Diffusions-Sperrschicht auf der NDI-Teilchenoberfläche die Reaktion der NCO-Gruppen gegenüber den aromatisch gebundenen $NH_2$-Gruppen stark desaktiviert ist. Es erfolgt auch bei 60-80°C keine Vorreaktion, die zu einem starken Viskositätsaufbau führt. Dieser so stabilisierte Reaktionsansatz kann in $H_2O$-Strahl-Vakuum entgast und nach bereits beschriebenen Arbeitstechnik zu Elastomeren verarbeitet werden.

Es wurden folgende mechanische Werte erhalten:

| | |
|---|---|
| Shore-Härte A | 91 |
| Belastung bei 100% MPa | 10,7 |
| Zugfestigkeit MPa | 15,2 |
| Bruchdehnung % | 550 |
| Weiterreißfestigkeit KN/m | 57,5 |
| Stoßelastizität % | 56 |

**Patentansprüche**

1.  Verfahren zur Herstellung von Harnstoff- und Biuretgruppen enthaltendem 1,5-Naphthylendiisocyanat, dadurch gekennzeichnet, daß in geschmolzener Form vorliegendes 1,5-Naphthylendiisocyanat mit 0,02 bis 0,5 Mol Wasser pro Mol Isocyanat in Abmischung mit der 1- bis 10-fachen Menge, berechnet pro Gewichtseinheit Wasser, eines mit Wasser mischbaren Lösungsmittels, das einen Siedebereich von 80°C bis 140°C aufweist oder eines mit Wasser im Siedebereich ein azeotropes Gasgemisch bildenden organischen Lösungsmittels umgesetzt wird.

2.  Verfahren gemäß Anspruch 1, dadurch gekennzeichnet, daß die Umsetzung mit Wasser in einem Temperaturbereich von 130° bis 160°C durchgeführt wird.

3.  Verfahren gemäß Anspruch 1 und 2, dadurch gekennzeichnet, daß ein Lösungsmittel verwendet wird, das einen Siedebereich von 100 bis 120°C aufweist.

4.  Verfahren gemäß einem der Ansprüche 1 bis 3, dadurch gekennzeichnet, daß 0,1 bis 0,25 Mol Wasser pro Mol Isocyanat eingesetzt werden.

5.  Verfahren gemäß einem der Ansprüche 1 bis 4, dadurch gekennzeichnet, daß die 2- bis 5-fache Menge, berechnet pro Gewichtseinheit Wasser, des organischen Lösungsmittels eingesetzt werden.

6.  Verfahren gemäß Anspruch 1, dadurch gekennzeichnet, daß die Umsetzung mit Wasser in einem Temperaturbereich von 130° bis 140°C durchgeführt wird.

**Claims**

1.  A process for the production of 1,5-naphthylene diisocyanate containing urea and biuret groups, characterized in that molten 1,5-naphthylene diisocyanate is reacted with 0.02 to 0.5 mol water per mol isocyanate in admixture with 1 to 10 times the quantity, per unit by weight of water, of a water-miscible solvent with a boiling range of 80°C to 140°C or an organic solvent which forms an azeotropic gas mixture with water in the boiling range.

2.  A process as claimed in claim 1, characterized in that the reaction is carried out with water at a temperature in the range from 130°C to 160°C.

3.  A process as claimed in claims 1 and 2, characterized in that a solvent with a boiling range of 100°C to 120°C is used.

4.  A process as claimed in any of claims 1 to 3, characterized in that 0.1 to 0.25 mol water is used per mol isocyanate.

5.  A process as claimed in any of claims 1 to 4, characterized in that 2 to 5 times the quantity, per unit by weight of water, of the organic solvent is used.

**6.** A process as claimed in claim 1, characterized in that the reaction with water is carried out at a temperature in the range from 130°C to 140°C.

**Revendications**

**1.** Procédé de préparation d'un 1,5-naphtylène-diisocyanate contenant des groupes urée et biuret, caractérisé en ce que l'on fait réagir le 1,5-naphtylène-diisocyanate à l'état fondu avec 0,02 à 0,5 mol d'eau par mole d'isocyanate en mélange avec 1 à 10 fois le poids d'eau d'un solvant organique miscible à l'eau ou formant un mélange gazeux azéotrope avec l'eau dans l'intervalle d'ébullition.

**2.** Procédé selon revendication 1, caractérisé en ce que la réaction avec l'eau est exécutée dans l'intervalle de température de 130 à 160°C.

**3.** Procédé selon revendication 1 et 2, caractérisé en ce que l'on utilise un solvant bouillant dans l'intervalle de 100 à 120°C.

**4.** Procédé selon l'une des revendications 1 à 3, caractérisé en ce que l'on met en oeuvre de 0,1 à 0,25 mol d'eau par mole d'isocyanate.

**5.** Procédé selon l'une des revendications 1 à 4, caractérisé en ce que l'on met en oeuvre le solvant organique en quantité de 2 à 5 fois le poids d'eau.

**6.** Procédé selon revendication 1, caractérisé en ce que la réaction avec l'eau est réalisée dans l'intervalle de température de 130 à 140°C.